# EUROPEAN PATENT APPLICATION

(11) **EP 2 977 045 A1**
(43) Date of publication of application: **27.01.2016**
(21) Application number: 15177490.8
(22) Date of filing: 20.07.2015
(51) Int. Cl.: A61K 31/135, A61K 31/27, A61K 31/38, A61K 31/421, A61K 31/445, A61K 31/704, A61K 31/138, A61K 31/197, A61K 31/4178, A61K 31/423, A61K 31/433, A61K 31/49, A61K 31/5513, A61P 21/02

(54) **ZALTOPROFEN AND MUSCLE RELAXANT COMBINATIONS**

(30) Priority: 21.07.2014 TR 201408575
(71) Applicant: Sanovel Ilac Sanayi ve Ticaret A.S., 34460 Istanbul (TR)
(72) Inventor: CIFTER, Ümit, 34460 Istanbul (TR); TÜRKYILMAZ, Ali, 34460 Istanbul (TR); ILDES ERDEM, Ayse, 34460 Istanbul (TR); YILDIRIM, Ediz, 34460 Istanbul (TR); KARABULUT, Tutku Ceren, 34460 Istanbul (TR)
(74) Representative: Sevinç, Erkan

(57) **Abstract**

The present invention relates to a novel pharmaceutical composition comprising zaltoprofen or a pharmaceutically acceptable salt thereof in combination with muscle relaxants with anti-inflammatory, analgesic and myorelaxant activity.

## Description

### Technical Aspect

The present invention relates to a novel pharmaceutical composition comprising zaltoprofen or a pharmaceutically acceptable salt thereof in combination with muscle relaxant drugs with anti-inflammatory, analgesic and myorelaxant activity.

More specifically, this invention relates to a pharmaceutical composition comprising zaltoprofen or a pharmaceutically acceptable salt thereof in combination with muscle relaxants with anti-inflammatory, analgesic and myorelaxant activity administrated orally, parenteraly, intramuscularly or topicaly in the form of a tablet, bilayer tablet, multilayer tablet, capsule, injectable preparat, suspension, syrup, sachet, ointment, cream or a gel form.

### Background of the Invention

Zaltoprofen is a propionic acid derivative, is a known NSAID (non-steroidal anti-inflammatory drug) with analgesic and anti-inflammatory activity. Its chemical structure is shown in the Formula I.

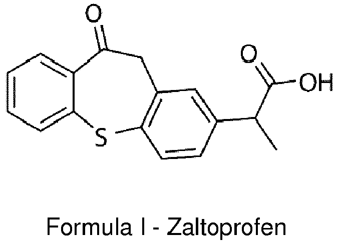

The chemical name of zaltoprofen is 2-(10-oxo-10,11-dihydrodibenzo[b,f]thiepin-2-yl)propanoic acid. It is a preferential COX-2 inhibitor. It selectively inhibits PGE2 (Prostaglandin E2) that mediates the pain pathway. It inhibits bradykinin-induced pain responses without interfering with the bradykinin receptors. It is used in musculoskeletal and joint disorders such as osteoarthritis and rheumatoid arthritis and other chronic inflammatory pain conditions or the treatment of lumbar pain, frozen shoulder, musculoskeletal pain, dental pain, post-operative pain, cervicobrachial syndrome, other pain and inflammatory conditions. It has also effect on post-surgery or post trauma chronic inflammation.

Muscle relaxants are used alone or in combination with analgesics in the management of musculoskeletal and neuromuscular disorders. There are two main types; centrally acting relaxants and directly acting relaxants.

Centrally acting relaxants generally have a selective action on the central nervous system (CNS) and are principally used for relieving painful muscle spasms or spasticity occurring in musculoskeletal and neuromuscular disorders. Spasms are sudden alternating contractions and relaxations or sustained contractions of muscle. Antispasmodic drugs are used to treat musculoskeletal conditions or inflamation, which includes back pain. Spasticity is defined as an upper motor neuron disorder, possibly caused by a conduction interruption in the nerve pathway. Antispastic drugs are primarily used to treat neurological disorders, such as cerebral palsy.

Tizanidine, dantrolene, thiocolchicoside and carisoprodol are known muscle relaxant agents used in the treatment of painful muscle spasms and spasticity occurring in musculoskeletal and neuromuscular disorders and for treating contractures and inflammatory conditions that affect the muscular system.

Tizanidine is an example for antispastic drugs. Its chemical structure is shown in Formula II.

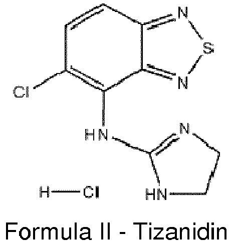

Tizanidine is a α₂-adrenergic agonist and acts mainly at spinal and supraspinal levels to inhibit excitatory interneurones. It is used for the symptomatic relief of spasticity associated with multiple sclerosis or with spinal cord injury or disease. The recommended dose of tizanidin is 2 mg, 4mg or 6 mg.

Dantrolene is also an antispastic drug indicated in controlling the manifestations of clinical spasticity resulting from upper motor neuron disorders (e.g., spinal cord injury, stroke, cerebral palsy, or multiple sclerosis). Its chemical structure is shown in Formula III.

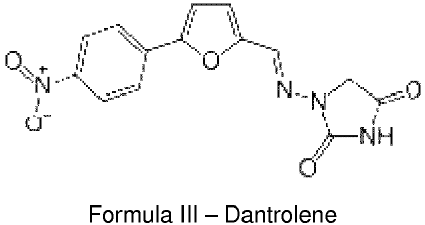

The recommended dose of dantrolene is 25 mg to 100 mg four times a day and at bedtime.

Thiocolchicoside is an antispasmodic drug that is a gamma-aminobutiric acid receptor agonist. Its chemical structure is shown in Formula IV.

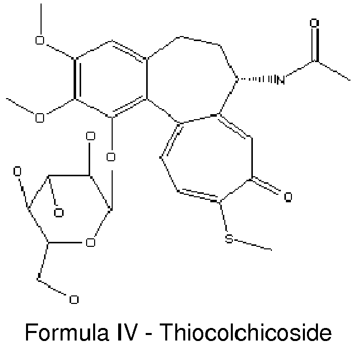

It has recently been shown that thiocoichicoside's activity can be ascribed to its ability of interacting with the strychnine-sensitive glycine receptors and therefore that compounds endowed with glycino-mimetic activity can be used in the rheumatologic-orthopedic field for their muscle relaxant properties.

The maximum recommended oral dose of Thiocolchicoside is 8 mg every 12 hours; treatment duration should be no more than 7 consecutive days. When given intramuscularly, the maximum dose should be 4 mg every 12 hours, for up to 5 days.

In addition, Carisoprodol is an antispasmodic drug indicated for the relief of discomfort associated with acute, painful musculoskeletal conditions. Its chemical structure is shown in Formula V.

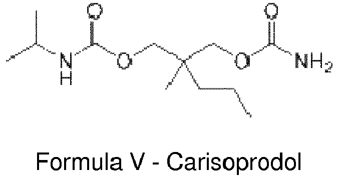

The recommended dose of Carisoprodol is 250 mg to 350 mg three times a day and at bedtime. The recommended maximum duration of Carisoprodol use is up to two or three weeks.

Muscle relaxants have been evaluated alone or in combination with conventional analgesics for the treatment of pain. Mixed and unpredictable results have been obtained in a pharmaceutical composition. But zaltoprofen has not previously been combined with muscle relaxants in a pharmaceutical composition for the treatment of inflammatory, pain and musculoskeletal diseases.

PCT application WO 86/03681 A1 , relates generally to novel pharmaceutical compositions of matter comprising one or more non-steroidal anti-inflammatory drugs other than aspirin, acetaminophen and phenacetin, in combination with at least one skeletal muscle relaxant, and optionally xanthine or a xanthine derivative, such as caffeine, and to methods of using said compositions in the treatment o a variety of skeletal muscle disorders including skeletal muscle spasm, certain orthopedic conditions, disk syndromes and low back pain.

United Kingdom patent application GB 2 197 198 A1 (Sandoz Ltd.) 03.11.1986, describes to novel pharmaceutical preparations comprising ibuprofen and tizanidine with analgesic and myotonolytic activity as well as to methods of inducing analgesia and of treating conditions associated with increased muscle tone.

It is well known that drugs used in the same therapeutic area or even for treating the same indication cannot always be combined *a priori* with the expectation of at least additive therapeutic effects. The scientific literature is full of examples wherein compounds of different classes, which are used to treat the same indications, cannot be combined into safe and efficacious dosage forms thereby resulting in incompatible drug combinations. The reasons for this unexpected lack of compatibility are varied; however, it is often found that the incompatible drug combinations result in increased side effects, unwanted drug interactions or additional side effects. More specifically, in the area of analgesia there are drug combinations that are contraindicated for some or all of these very same reasons.

Conventional analgesic and myorelaxant therapy generally involves administration of a pharmaceutical composition containing one or more different analgesic and muscle relaxant drugs. However, not all combinations of analgesic drugs and muscle relaxant drugs are more suitable, in terms of safety or efficacy, than the administration of a single product.

Thus, there is a need in the art for a pharmaceutical composition or a dosage form comprising a combination of a zaltoprofen and a muscle relaxant, in particular tizanidine, thiocolchicoside, dantrolene or carisopradol.

### Detailed description of the invention

The present invention relates to a pharmaceutical composition comprising zaltoprofen or a pharmaceutically acceptable salt thereof in combination with muscle relaxant drugs.

In one embodiment, pharmaceutical composition comprising zaltoprofen or a pharmaceutically acceptable salt thereof in combination with muscle relaxants with anti-inflammatory, analgesic and myorelaxant activity administrated orally, parenteraly, intramuscularly or topicaly in the form of a tablet, bilayer tablet, multilayer tablet, capsule, injectable preparat, suspension, syrup, sachet, ointment, cream or a gel.

According to one embodiment, the present composition is in the form of a tablet, bilayer tablet, multilayer tablet or a capsule.

Novel pharmaceutical composition in the form of a tablet or a capsule administrated orally may provide a significant advance in the available treatments. Such combination therapy may also provide for therapeutic improvements owing to the potential synergistic effect provided by the combination.

Muscle relaxants suitable for use in the composition of the present invention are selected from the group comprising thiocolchicoside, carisoprodol, tizanidine, dantrolene, flavoxate, cyclobenzaprine, baclofen, diazapem, metaxalone, methocarbamol, metocurine iodide, succinylcholine, orphenadrine, quinine, chlorzoxazone. Muscle relaxants used in the composition of this present invention is an antispasmodic drug or an antispastic drug.

In one embodiment, antispasmodic drugs are selected from the group comprising, thiocolchicoside, carisoprodol, flavoxate, cyclobenzaprine, metaxalone, metocurine iodide, orphenadrine or chlorzoxazone. Preferably they are thiocolchicoside or carisoprodol or pharmaceutically acceptable salts thereof. More preferably the antispasmodic drug is thiocolchicoside.

In one embodiment, antispastic drugs are selected from the group comprising, tizanidine, dantrolene, baclofen, diazapem, methocarbamol, succinylcholine, quinine. Preferably they are tizanidine or dantrolene or pharmaceutically acceptable salts thereof. More preferably the antispastic drug is tizanidine.

According to one embodiment, zaltoprofen or a pharmaceutically acceptable salt thereof is present in an amount of between 10 - 90%, preferably 15 - 50% and more preferably 25 - 35% by weight of total composition.

According to one embodiment, thiocolchicoside or a pharmaceutically acceptable salt thereof is present in an amount of between 1 - 10%, preferably 2 - 8%, more preferably 2 - 4% by weight of total composition.

According to one embodiment, tizanidine or a pharmaceutically acceptable salt thereof is present in an amount of between 0.5 - 10%, preferably 0.5 - 8%, more preferably 0.5 - 4% weight of total composition.

According to one embodiment, dantrolene or a pharmaceutically acceptable salt thereof is present in an amount of between 2 - 90%, preferably 3 - 50%, more preferably 5 - 30% weight of total composition.

According to one embodiment, carisoprodol or a pharmaceutically acceptable salt thereof is present in an amount of between 2 - 90%, preferably 10 - 80%, more preferably 30 - 50% weight of total composition.

Further embodiment of the present invention provides a pharmaceutical composition comprising zaltoprofen in combination with muscle relaxants in particular tizanidine, thiocolchicoside, dantrolene or carisopradol in the treatment of painful muscle spasms associated with static and functional disorders of vertebra or occurred in post-operations of osteoarthritis, pain and inflammatory symptoms associated with tissue trauma, degenerative vertebra diseases as torticollis, dorsalgy, lombalgy, disk hernia, neurologic and traumatic disorders associated with spasticity.

The main challenges when combining two or more molecules in the same pharmaceutical form are (a) to guarantee the chemico-physical compatibility between the different active ingredients and/or between the active ingredients and the excipients used; and (b) to insure the therapeutical compatibility between the two active ingredients regarding their pharmacokinetic and/or pharmaceutical properties in order that the posology of the combined composition allows to obtain safe and efficient plasma levels of both pharmacological agents.

According to these main challenges above, the pharmaceutical composition comprising zaltoprofen in combination with muscle relaxants in particular tizanidine, thiocolchicoside, dantrolene or carisopradol have an additive analgesic effect in relief of postoperative pain and provide greater analgesia with the results in a lower incidence of side effects according to *priori.* These pharmaceutical combinations are administrated orally, parenteraly, intramuscularly or topicaly.

The pharmaceutical compositions of the invention include tablets, bilayer tablet, multilayer tablet, capsules, injectables, suspensions, syrups, sachets, ointments, creams or gels can be made in accordance with methods that are standard in the art. Examples of oral dosage forms include tablets (including compressed, coated or uncoated), capsules, hard or soft gelatin capsules, pellets, pills, powders, granules, elixirs, tinctures, colloidal dispersions, dispersions, effervescent compositions, films, sterile solutions or suspensions, syrups or emulsions

According to one embodiment, the combination of a zaltoprofen with tizanidine, thiocolchicoside, dantrolene or carisoprodol is in the form of a tablet or a capsul. Moreover, it may be granulated by methods such as, dry granulation, low- or high-shear granulation, wet granulation or fluidized-bed granulation. Low-shear granulation, high-shear granulation, wet granulation and fluidized-bed granulation generally produce harder, less friable tablets.

In one embodiment, pharmaceutical composition of this present invention further comprises at least one pharmaceuticlly acceptable excipient. According to this embodiment, at least one pharmaceuticlly acceptable excipient is selected from a group comprising binders, diluents, disintegrants, lubricants and glidants or mixtures thereof.

According to this embodiment, binders used in the present composition are selected from the group comprising hydroxypropyl cellulose, hydroxyethylmethyl cellulose, povidone, carbomers, carboxymethylcellulose sodium, cellulose acetate phthalate, chitosan, copovidone, corn starch, pregelatinized starch, dextrates, dextrin, dextrose, ethylcellulose, gelatin, glyceryl behenate, guar gum, hydrogenated vegetable oil type I, hydroxyethyl cellulose, hydroxypropyl starch, hypromellose, liquid glucose, magnesium aluminum silicate, maltodextrin, maltose, methylcellulose, pectin, poloxamer, polycarbophil, polydextrose, polyethylene oxide, polymethacrylates, sodium alginate, stearic acid, sucrose or mixtures thereof. Preferably, binder is hydroxypropyl cellulose.

In a further embodiment, hydroxypropyl cellulose (HPC) is used as a binder in the composition of the present invention to improve tabletting characteristics. Zaltoprofen and muscle relaxants used in the composition have different flowability, solubility and viscosity characteristics. It makes the granulation more difficult. Moreover, zaltoprofen and muscle relaxants used in the composition are moisture sensitive. In this invention, to overcome these problems, HPC is used and it has been suprisingly found that HPC provides both better tableting characteristics and stability of the composition.

According to one embodiment, diluents used in the present composition are selected from the group comprising lactose, mannitol, microcrystalline cellulose, starch, sodium carbonate, sodium bicarbonate, calcium carbonate, sucrose and mixtures thereof. Preferably, diluent is lactose.

According to one embodiment, disintegrants used in the present composition are selected from the group comprising microcristalline cellulose, croscarmellose sodium, sodium starch glycollate, crospovidone, starch and their mixtures thereof. Preferably, disintegrant is microcristalline cellulose, croscarmellose sodium or mixtures thereof.

According to one embodiment, lubricants used in the present composition are selected from the group comprising of magnesium stearate, stearic acid, colloidal silicon dixode, collodial anhydrous silica, talc, sodium stearil fumarate and mixtures thereof. Preferably, lubricant is magnesium stearate or stearic acid.

According to one embodiment, glidants used in the present composition are selected from the group comprising of colloidal silicon dioxide, talc, aluminium silicate or mixtures thereof; preferably glidant is colloidal silicon dioxide.

Coating agents may include but not limited OpadryTM derivatives (such as opadry yellow (20A22418) and opadry II), aminoalkyl metacrylate, carboxymethylcellulose calcium, carboxymethylcellulose sodium, carnauba wax, cellulose acetate, cellulose acetate phthalate, ceresin, cetyl alcohol, chitosan, ethylcellulose, fructose, gelatin, glycerin, glyceryl behenate, glyceryl palmitostearate, hydroxyethyl cellulose, hydroxyethylmethyl cellulose, hydroxypropyl cellulose, hypromellose, hypromellose phthalate, isomalt, liquid glucose, maltitol, maltodextrin, methylcellulose, microcrystalline wax, paraffin, poloxamer, polydextrose, polyethylene oxide, poly-DL-(lactic acid), polyvinyl acetate phthalate, potassium chloride, shellac, shellac with stearic acid, sucrose, surface color agents, titanium oxide, tributyl citrate, triethyl citrate, vanillin, white wax, xylitol, yellow wax, zein, dimethylaminoethyl methacrylate, butyl methacrylate and methyl methacrylate (Eudragit E 100) (Poly(butyl methacrylate-co-(2- demethylaminoeethyl)methacrylate-co-methyl methacrylate)) or mixture of polyethylene glycol and polyvinyl alcohol (Kollicoat IR) and their copolymers, hydroxypropyl methyl cellulose (HPMC), polyethyleneglycol (PEG), polivinylpyrrolidon (PVP), polyvinyl alcohol (PVA), vinylpyrrolidone-vinyl acetate copolymer (PVP- PVAc) and pigments, titanium dioxide, dyes and iron oxide and talc or mixtures thereof.

In this present invention, zaltoprofen and muscle relaxant compositions have been designed, comprising of the following:
- 10 - 90% by weight of zaltoprofen or pharmaceutically acceptable salt thereof,
- 1 - 10% by weight of thiocolchicoside or pharmaceutically acceptable salt thereof,
- 1 - 60 % by weight of lactose,
- 5 - 80 % by weight of microcrystalline celluose,
- 0.5 - 5 % by weight of croscarmellose sodium
- 0.1 - 50 % by weight of hydroxypropyl cellulose
- 0.01 - 5 % by weight of colloidal silicon dioxide,
- 0.1 - 5 % by weight of magnesium stearate,
- water and,
- optionally, coating.

- 10 - 90% by weight of zaltoprofen or pharmaceutically acceptable salt thereof,
- 0.5 - 10% by weight of tizanidin or pharmaceutically acceptable salt thereof,
- 1 - 60 % by weight of lactose,
- 5 - 80 % by weight of microcrystalline celluose,
- 0.1 - 50 % by weight of hydroxypropyl cellulose
- 0.01 - 5 % by weight of colloidal silicon dioxide,
- 0.1 - 5 % by weight of stearic acid,
- water and,
- optionally, coating.

- 10 - 90% by weight of zaltoprofen or pharmaceutically acceptable salt thereof,
- 2 - 90% by weight of dantrolene or pharmaceutically acceptable salt thereof,
- 1 - 60 % by weight of lactose,
- 5 - 80 % by weight of microcrystalline celluose,
- 0.1 - 50 % by weight of hydroxypropyl cellulose
- 0.01 - 5 % by weight of colloidal silicon dioxide,
- 0.1 - 5 % by weight of stearic acid,
- water and,
- optionally, coating.
and
- 10 - 90 % by weight of zaltoprofen or pharmaceutically acceptable salt thereof,
- 2 - 90% by weight of carisoprodol or pharmaceutically acceptable salt thereof,
- 1 - 60 % by weight of lactose,
- 5 - 80 % by weight of microcrystalline celluose,
- 0.5 - 5 % by weight of croscarmellose sodium
- 0.1 - 50 % by weight of hydroxypropyl cellulose
- 0.01 - 5 % by weight of colloidal silicon dioxide,
- 0.1 - 5 % by weight of magnesium stearate,
- water and,
- optionally, coating.

### Example 1

| **Ingredient** | **(%) amount** |
|---|---|
| zaltoprofen | 25.0 - 35.0 |
| thiocolchicoside | 2.0 - 4.0 |
| lactose | 5.0 - 50.0 |
| microcrystalline celluose PH 101 | 5.0 - 50.0 |
| croscarmellose sodium | 2.0 - 3.0 |
| hydroxypropyl cellulose LF | 0.5 - 20.0 |
| colloidal silicon dioxide | 0.05 - 2.0 |
| magnesium stearate | 0.1 - 5.0 |
| coating | 0.01 - 3.0 |
| water | q.s. |

The process of the composition is carried out as follows: Zaltorprofen, thiocolchicoside, lactose, croscarmellose sodium and microcrystalline celluose (PH 101) are taken into fluid bed dryer and mixed. Solution of hydroxypropyl cellulose LF is prepared and granulation is performed by spraying this solution on the mixture. Granules are dried and sieved. Collodial silicon dioxide is added to granules and mixed. Magnesium stearate is added to this mixture and mixed again. Total mixture is pressed into the tablets. Tablets are coated with Opadry yellow (20A22418).

### Example 2

| **Ingredient** | **(%) amount** |
|---|---|
| zaltoprofen | 25.0 - 35.0 |
| tizanidin hydrochloride | 0.5 - 4.0 |
| lactose | 5.0 - 50.0 |
| microcrystalline celluose PH 101 | 5.0 - 50.0 |
| hydroxypropyl cellulose LF | 0.5 - 20.0 |
| colloidal silicon dioxide | 0.05 - 2.0 |
| stearic acid | 0.1 - 5.0 |
| coating | 0.01 - 3.0 |
| water | q.s. |

The process of the composition is carried out as follows: zaltoprofen, lactose, hydroxypropyl cellulose and microcrystalline cellulose (PH 101) are sieved and mixed. Wet granulation is performed. Then, granules are dried and sieved. Tizanidin hydrochloride, stearic acid and colloidal silicon dioxide are sieved and added to granules then mixed again. Total mixture is pressed into tablets. Tablets are coated with Opadry II.

### Example 3

| **Ingredient** | **(%) amount** |
|---|---|
| zaltoprofen | 25.0 - 35.0 |
| dantrolene | 5.0 - 30.0 |
| lactose | 5.0 - 50.0 |
| microcrystalline celluose PH 101 | 5.0 - 50.0 |
| hydroxypropyl cellulose LF | 0.5 - 20.0 |
| colloidal silicon dioxide | 0.05 - 2.0 |
| stearic acid | 0.1 - 5.0 |
| coating | 0.01 - 3.0 |
| water | q.s. |

The process of the composition is carried out as follows: Zaltoprofen, lactose, hydroxypropyl cellulose LF and microcrystalline celluose (PH 101) are sieved and mixed. Wet granulation is performed. Then granules are dried and sieved. Dantrolene, stearic acid and colloidal silicon dioxide are sieved and mixed with granules. Total powder pressed into tablets. Tablets are coated with Opadry II.

### Example 4

| **Ingredient** | **(%) amount** |
|---|---|
| zaltoprofen | 25.0 - 35.0 |
| carisoprodol | 30.0 - 50.0 |
| lactose | 5.0 - 50.0 |
| microcrystalline celluose PH 101 | 5.0 - 50.0 |
| croscarmellose sodium | 2.0 - 3.0 |
| hydroxypropyl cellulose LF | 0.5 - 20.0 |
| colloidal silicon dioxide | 0.05 - 2.0 |
| magnesium stearate | 0.1 - 5.0 |
| coating | 0.01 - 3.0 |
| water | q.s. |

The process of the composition is carried out as follows: Zaltorprofen, carisoprodol, lactose, croscarmellose sodium and microcrystalline celluose (PH 101) are taken into fluid bed dryer and mixed. Solution of hydroxypropyl cellulose LF is prepared and granulation is performed by spraying this solution on the mixture. Granules are dried and sieved. Collodial silicon dioxide is added to granules and mixed. magnesium stearate is added to this mixture and mixed again. Total mixture is pressed into the tablets. Tablets are coated with Opadry yellow (20A22418).

## Claims

1. A pharmaceutical composition comprising zaltoprofen or a pharmaceutically acceptable salt thereof in combination with muscle relaxant drugs.

2. The pharmaceutical composition according to claim 1, wherein the muscle relaxant drug is an antispasmodic drug or an antispastic drug.

3. The pharmaceutical composition according to claim 2, wherein antispasmodic drugs are selected from the group comprising thiocolchicoside, carisoprodol, flavoxate, cyclobenzaprine, metaxalone, metocurine iodide, orphenadrine or chlorzoxazone. Preferably, they are thiocolchicoside or carisoprodol or pharmaceutically acceptable salts thereof.

4. The pharmaceutical composition according to claim 2, wherein antispastic drugs are selected from the group comprising tizanidine, dantrolene, baclofen, diazapem, methocarbamol, succinylcholine, quinine. Preferably, they are tizanidine or dantrolene or pharmaceutically acceptable salts thereof.

5. The pharmaceutical composition according to claim 1, 2 or 3, antispasmodic drug is thiocolchicoside.

6. The pharmaceutical composition according to claim 1, 2 or 4, antispastic drug is tizanidine.

7. The pharmaceutical composition of claim 1, wherein zaltoprofen or a pharmaceutically acceptable salt thereof is present in an amount of between 10 - 90%, preferably 15 - 50% and more preferably 25 - 35% by weight of total composition.

8. The pharmaceutical composition of claim 5, wherein thiocolchicoside or a pharmaceutically acceptable salt thereof is present in an amount of between 1 - 10%, preferably 2 - 8%, more preferably 2 - 4% by weight of total composition.

9. The pharmaceutical composition of claim 6, wherein tizanidine or a pharmaceutically acceptable salt thereof is present in an amount of between 0.5 - 10%, preferably 0.5 - 8%, more preferably 0.5 - 4% weight of total composition.

10. The pharmaceutical composition of claim 1 or 3, wherein carisoprodol or a pharmaceutically acceptable salt thereof is present in an amount of between 2 - 90%, preferably 10 - 80%, more preferably 30 - 50% weight of total composition.

11. The pharmaceutical composition of claim 1 or 4, wherein dantrolene or a pharmaceutically acceptable salt thereof is present in an amount of between 2 - 90%, preferably 3 - 50%, more preferably 5 - 30% weight of total composition.

12. The pharmaceutical composition according to any preceding claims, further comprising at least one pharmaceuticlly acceptable excipient.

13. The pharmaceutical composition according to claims 12, wherein at least one pharmaceuticlly acceptable excipient is selected from a group comprising binders, diluents, disintegrants, lubricants and glidants or mixtures thereof.

14. The pharmaceutical composition according to claim 13, wherein binders are selected from the group comprising hydroxypropyl cellulose, hydroxyethylmethyl cellulose, povidone, carbomers, carboxymethylcellulose sodium, cellulose acetate phthalate, chitosan, copovidone, corn starch, pregelatinized starch, dextrates, dextrin, dextrose, ethylcellulose, gelatin, glyceryl behenate, guar gum, hydrogenated vegetable oil type I, hydroxyethyl cellulose, hydroxypropyl starch, hypromellose, liquid glucose, magnesium aluminum silicate, maltodextrin, maltose, methylcellulose, pectin, poloxamer, polycarbophil, polydextrose, polyethylene oxide, polymethacrylates, sodium alginate, stearic acid, sucrose or mixtures thereof, preferably, binder is hydroxypropyl cellulose (HPC).

15. The pharmaceutical composition of any preceding claims, wherein said pharmaceutical composition is administrated orally, parenteraly, intramuscularly or topicaly.

16. The pharmaceutical composition of any preceding claims, wherein said pharmaceutical composition is in the form of a tablet, bilayer tablet, multilayer tablet, capsule, injectable preparat, suspension, syrup, sachet, ointment, cream or a gel.
